# EUROPEAN PATENT APPLICATION

(11) **EP 4 086 244 A1**
(43) Date of publication of application: **09.11.2022**
(21) Application number: 22382257.8
(22) Date of filing: 18.03.2022
(51) Int. Cl.: C07D 333/08

(54) **METHOD FOR PRODUCING TETRAHYDROTHIOPHENE FROM FURFURAL**

(30) Priority: 06.05.2021 ES 202130409
(71) Applicant: Enagas Emprende S.L., 28005 Madrid (ES)
(72) Inventor: DE LA FUENTE GONZÁLEZ, ROBERTO, MADRID (ES); ALADID GARCÍA, ANA, Madrid (ES); CAMPOS MARTÍN, JOSÉ MIGUEL, Madrid (ES); MORALES DE LA ROSA, SILVIA, Madrid (ES); MÁRQUEZ MEDINA, Mª DOLORES, Madrid (ES)
(74) Representative: Pons

(57) **Abstract**

The present invention relates to a new method for producing tetrahydrothiophene (THT) characterised in that it is produced from furfural, for use as an additive in Natural Gas, biomethane and hydrogen, from raw material of renewable origin, specifically from products that are produced from biomass.

## Description

The present invention relates to a new method for producing tetrahydrothiophene (THT) characterised in that it is produced from furfural for use thereof as an odourising additive in Natural Gas, Biomethane and H₂, from raw material of renewable origin, specifically from products that are produced from biomass.

### BACKGROUND OF THE INVENTION

Town and coke oven gases previously used for public gas supply contained strong odour components and, therefore, had a strong odour of their own, such that the outgoing gas could be easily perceived.

Natural gas is mainly made up of methane (typical methane contents are in the range of 50 to 99% by weight, most often in the range of 60 to 99% by weight and usually 80 to 99% by weight) and may additionally contain, depending on the origin, different proportions of ethane, propane and hydrocarbons with higher molecular weight. Natural gas H (H = High) has a methane content of 87 to 99.1% by volume, natural gas L (L = Low) generally contains 79.8 to 87% by volume of methane.

Due to the high degree of purity thereof, the gas currently used in the public network, normally obtained from natural gas, is almost odourless in itself. If leaks are not detected in time, rapidly explosive gas/air mixtures form with a high risk potential.

Gas odourisation is understood as the addition of substances with an intense odour acting as a warning or alarm substance (odourising agents) to gases that do not have a significant odour of their own, in other words, to otherwise essentially or completely odourless gases.

For safety reasons, the gas is odourised by adding strong-smelling substances. Thus, in Germany for example, it is stipulated that all gases that do not have a sufficient odour of their own and are distributed in the public gas supply are odourised according to DVGW worksheet G 280 (DVGW = German Association of the Gas and Water Industry, registered association, of "Deutscher Verein des Gas- und Wasserfaches e.V."). These odourising agents should also be detectable in high dilution and, as desired, cause an association of alarm in humans due to their extremely unpleasant odour. The odourising agent must not only smell unpleasantly and unmistakably, but above all clearly represent a warning smell. Therefore, the odour of the odourised gas must not be common to humans in everyday life, for example, from the kitchen and home. In Germany, approximately 90% of useful gas is currently odourised with tetrahydrothiophene (THT) (12 - 25 mg/m³); moreover, odourisation with mercaptans is also common.

It may be practical to add a larger amount of odourising agent to the gas over an extended period of time. In the so-called shock odourisation, one to ten times the amount of odourising agent is introduced into the gas compared to usual odourisation. Shock odourisation is applied, for example, in the commissioning of new networks or pipe sections to quickly reach the minimum concentration of odourising agent, or also to check small leaks in the gas installation.

THT alone is excellently suitable for effective gas odourisation. However, THT is conventionally obtained from petroleum-derived products or of fossil origin. Tetrahydrothiophene is prepared by means of reacting tetrahydrofuran with hydrogen sulphide. This vapour phase reaction is catalysed by alumina and other heterogeneous acid catalysts. Said tetrahydrofuran is produced from the acid-catalysed dehydration of 1,4-butanediol. The method is similar to the production of diethyl ether from ethanol. Butanediol is obtained from the condensation of acetylene with formaldehyde, followed by hydrogenation. A process was also developed to produce THF by means of the oxidation of n-butane to raw maleic anhydride, followed by catalytic hydrogenation. A third important industrial route involves the hydroformylation of the allyl alcohol followed by hydrogenation to 1,4-butanediol.

### DESCRIPTION OF THE INVENTION

In a first aspect, the present invention relates to a method for producing THT characterised in that it is obtained from furfural and comprises the following steps
a) decarbonylating the furfural in the presence of a catalyst comprising Pd between 0.1% and 10% by weight, on alumina (Al₂O₃) doped with a metal selected from among alkaline and alkaline earth metal, at a temperature between 300 °C and 400 °C and a pressure between 1 bar and 20 bar, in the presence of H₂;
b) hydrogenating the furan obtained in step (a) in the presence of a palladium catalyst between 0.1% and 5% by weight, supported on alumina, at a temperature between 80 and 180 °C, and a pressure between 5 bar and 50 bar in the presence of an excess of hydrogen; and
c) sulphurising the THF obtained in step (b) with H₂S in a stoichiometric amount or less in the presence of an alumina-silica catalyst, wherein the silica content in the catalyst is between 0.1 and 10% by weight, at a temperature between 330 °C and 450 °C and a pressure between 1 bar and 10 bar.

The advantages associated with the method are that with the alumina that is doped in step (a) it causes the catalyst to be more stable and have a higher conversion and selectivity at long reaction times. After one hour of reaction, furfural conversion is 99.9% and selectivity to furan is 96.5%, this behaviour is maintained at least after 4.5 h of reaction, furan conversion 99.9% and selectivity to furan 96.0%, making it possible to work with high values of conversion and selectivity to furan for prolonged periods.

Another advantage is to use the alumina support for the catalyst of step (b), since in case of deactivation of the catalyst it can be easily regenerated by means of calcination, while a carbon support, widely used in the state of the art, cannot be regenerated by means of calcination because the support (carbon) is burnt.

Another advantage of step c) using values equal to or slightly less than the stoichiometric ratio between the H₂S and the THF, is that at the outlet of the reactor the concentration of H₂S is very low, and therefore the separation of the products and the treatment of the effluents are much simpler.

Additionally, another advantage of the method is the use of raw material of renewable origin from any biomass and, therefore, contributing to the objectives of decarbonisation and energy transition by replacing the traditional model of fossil origin (the production process is different since the raw material is different). For example and without limitation, furfural is produced from 2nd generation lignocellulosic biomass, specifically it is produced from C5 sugars so, it thereby contributes to the circular economy and the objectives of the energy transition by favouring decarbonisation and innovating and giving it a new industrial use.

In a preferred embodiment of the method, the alkali and alkaline earth metal of step (a) with which the alumina is doped is selected from sodium (Na), potassium (K), rubidium (Rb), magnesium (Mg), calcium (Ca) and barium (Ba). In a more preferred embodiment, the metal is potassium. In another more preferred embodiment, the metal is calcium. The addition of these metals in low amounts (< 15% by weight) produces a more stable behaviour of the catalyst for long periods of time, in addition, a decrease in the catalytic activity of palladium is not observed when the expectation is that, when an alkali metal is introduced, this acts as catalyst poison and performance would be expected to drop. In other words, what it does is inhibit secondary reactions and prevents the formation of by-products, increasing both the conversion and selectivity of the step.

In another preferred embodiment of the method, the pressure of step (a) is between 8 and 15 bar.

In another preferred embodiment of the method, the furfural / hydrogen molar ratio of step (a) is between 0.2:1 and 3:1.

In another preferred embodiment of the method, the pressure of step (c) is between 1 bar and 1.5 bar. The conversion of THT and selectivity to THT are improved by around 4% conversion and 2% selectivity with the indicated working pressure compared to a pressure between 2 bar and 5 bar. In addition, it is advisable to work at pressures close to the atmospheric pressure because the investment in equipment and energy costs is lower.

A second aspect of the present invention is a gas odourisation method comprising the following steps:
i. producing the THT according to the method described in claims 1 to 7; and
ii. mixing the THT produced in (i) with a gas selected from natural gas, biomethane and hydrogen.

Throughout the description and the claims, the word "comprises" and its variants do not intend to exclude other technical features, additives, components or steps. For those skilled in the art, other objects, advantages and features of the invention may be partially deduced from both the description and the embodiment of the invention. The following examples and figures are provided by way of illustration and are not intended to limit the present invention.

### BRIEF DESCRIPTION OF THE FIGURES

**Fig. 1****.** Diagram of Furfural to Furan reaction.
**Fig. 2****.** Conversion and selectivity of the Furfural to Furan reaction in the presence of a catalyst with 1% by weight of palladium stabilised with potassium.
**Fig. 3****.** Diagram of Furan to THF reaction.
**Fig. 4****.** Diagram of THF to THT reaction.
**Fig. 5****.** Conversion and selectivity of the reaction of THF to THT with different catalysts.
**Fig. 6****.** Conversion and selectivity of the reaction of THF to THT at different pressures.

### EXAMPLES

Next, the invention will be illustrated by means of assays carried out by the inventors that demonstrate the effectiveness of the product of the invention.

### Example 1

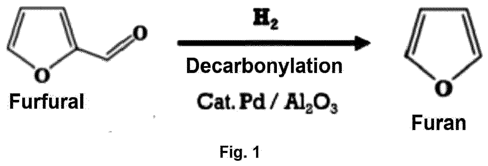

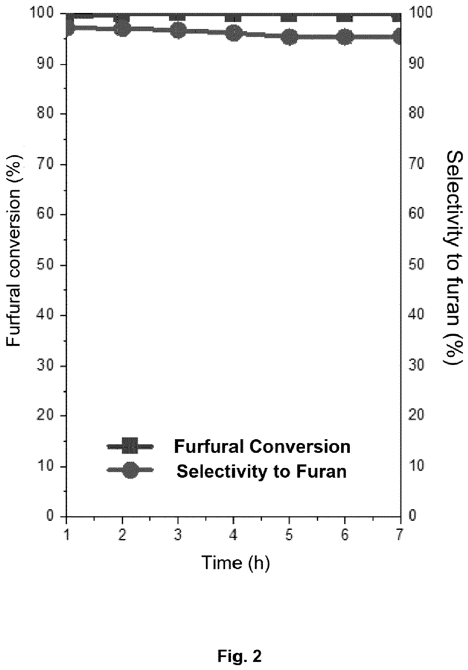

Furfural decarbonylation reaction for producing furan (Figure 1):
The decarbonylation catalyst uses a catalyst with 1% by weight of palladium supported on potassium-doped alumina (IBERCAT S. L., IBC-DCF-002). This catalyst was tested in the decarbonylation reaction of furfural to furan. 7.4 g of catalyst are placed in the centre of a tubular steel reactor with an inner diameter of 17.5 mm. The catalyst is activated under a stream of hydrogen diluted in nitrogen at atmospheric pressure with a temperature of 330 °C. Next, the pressure is raised to a pressure of 10 bar, the hydrogen flow is adjusted to 50 mln/min and the furfural feed (0.37 ml/min) is started. The products are collected at the outlet of the reactor. After one hour of reaction, furfural conversion is 99.9% and the selectivity to furan is 96.5%, this behaviour is maintained after 4.5 h of reaction, furan conversion 99.9% and selectivity to furan 96.0%. The catalyst used is doped with potassium, which makes the catalyst very stable over time, being able to be used with very high conversion and selectivity values for more than 4.5 h, when the expectation is that, when an alkali metal is introduced, this acts as catalyst poison and performance would be expected to drop. A similar behaviour is maintained for more than 24 h of reaction, as can be observed in Figure 2.

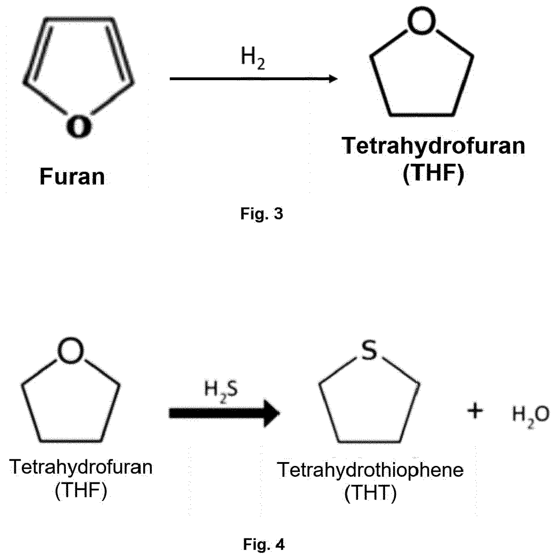

Furan hydrogenation reaction for producing Tetrahydrofuran (Figure 3):
2 g of 0.5 % by weight of palladium on alumina catalyst are placed in the centre of a tubular steel reactor with an inner diameter of 17.5 mm. The catalyst is activated under a stream of hydrogen diluted in nitrogen at atmospheric pressure with a temperature of 140 °C. Next, the pressure is raised to a pressure of 20 bar, the hydrogen flow is adjusted to 225 mln/min and the furan feed (0.1 ml/min) is started. The products are collected at the outlet of the reactor. Furan conversion is 99.8% and selectivity to THF is 99.5%.

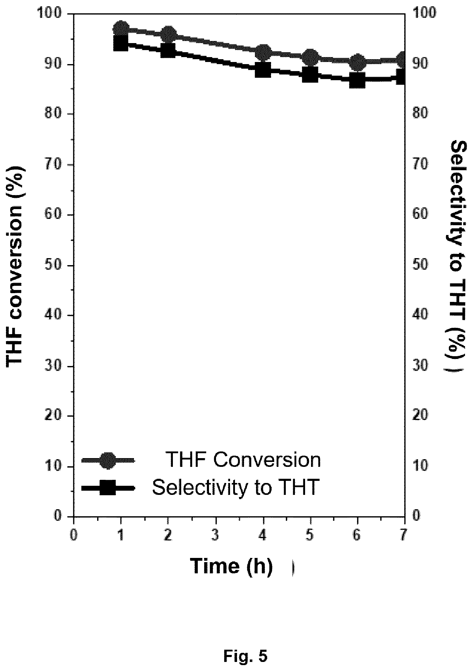

Sulphuration reaction of THF to THT (Figure 4):
6 g of alumina-silica catalyst (3.5 % by weight of silica) (SaintGobaint-Norpro SA69227) are placed in the centre of tubular steel reactor with an inner diameter of 17.5 mm. The catalyst is activated under a stream of nitrogen at atmospheric pressure with a temperature of 400 °C. Next, the temperature is lowered to 350 °C and the flow of hydrogen sulphide is adjusted to 45 mln/min and the THF feed (0.16 ml/min) is started. The products are collected at the outlet of the reactor. The conversion of THF is 100% and the selectivity to tetrahydrothiophene (THT) is 98% at the beginning of the reaction (Figure 5) and remains above 85% until after 6 hours.

The catalysts used in this reaction are stable acid solids at the reaction temperatures, and which can be easily regenerated. The catalysts that work best are based on alumina slightly modified with silica.

### Example 2

Comparative example with respect to the furfural decarbonylation reaction step for producing furan.

A 1% by weight of palladium catalyst supported on unstabilised alumina (Acros Organics ref. 195080500) is used, which is not the catalyst of this invention. The reaction conditions are the same as those used in example 1. After 1 h of reaction, the furfural conversion is 96.3% and the selectivity is 96.2%. After 4.5 h of reaction, the conversion value remains high at 95.4%, but the selectivity drops drastically to 51.8%; however, with the stabilised catalyst (which corresponds to this invention) for the same reaction time, the conversion and selectivity are much higher and similar to the initial ones (see example 1).

### Example 3

Comparative example with respect to the furfural decarbonylation reaction step for producing furan.

A palladium catalyst supported on undoped alumina (Alfa Aesar ref. 041825) is used, which is not the catalyst of this invention. The reaction conditions are the same as those used in example 1. After 1 h of reaction, the furfural conversion is 94.1% and the selectivity is 39.5%. After 4.5 h of reaction, the conversion value remains high at 92.5%, but the selectivity drops to 27.4%.

### Example 4

Pressure influence example in the step of the sulphuration reaction of THF to THT.

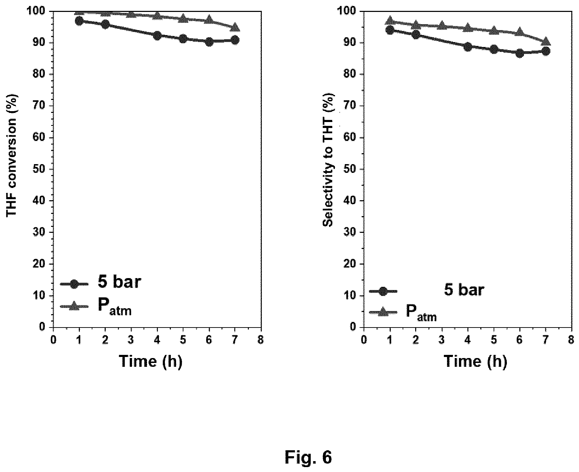

A comparison is made following example 1, the sulphuration reaction of THF to THT, wherein 6 g of 3.5% alumina-silica catalyst at 350 °C and a pressure of 5 bar, the THF conversion is 97% and the selectivity to THT is 94%. The operation is repeated, but the pressure is 1 bar, the conversion of THF is 99.9% and the selectivity to THT is 97%, as can be seen in Figure 6.

## Claims

1. A method for producing THT **characterised in that** it is produced from furfural and comprises the following steps
a) decarbonylating the furfural in the presence of a catalyst comprising Pd between 0.1 % and 10% by weight, on alumina (AI2Os) doped with a metal selected from among alkaline and alkaline earth metal, at a temperature between 300 °C and 400 °C and a pressure between 1 bar and 20 bar, in the presence of H₂;
b) hydrogenating the furan obtained in step (a) in the presence of a palladium catalyst between 0.1% and 5% by weight, supported on alumina, at a temperature between 80 and 180 °C, and a pressure between 5 bar and 50 bar in the presence of an excess of hydrogen; and
c) sulphurising the THF obtained in step (b) with H₂S in a stoichiometric amount or less in the presence of an alumina-silica catalyst, wherein the silica content in the catalyst is between 0.1 and 10% by weight, at a temperature between 330 °C and 450 °C and a pressure between 1 bar and 10 bar.

2. The method according to claim 1, wherein the alkali and alkaline earth metal of step (a) with which the alumina is doped is selected from sodium, potassium, rubidium, magnesium, calcium and barium.

3. The method according to claim 2, wherein the alkali metal and alkaline earth metal of step (a) with which the alumina is doped is potassium.

4. The method according to claim 2, wherein the alkali and alkaline earth metal of step (a) with which the alumina is doped is calcium.

5. The method according to any of claims 1 to 4, wherein the pressure of step (a) is between 5 bar and 15 bar.

6. The method according to any of claims 1 to 4, wherein the furfural / hydrogen molar ratio of step (a) is between 0.1:1 and 3:1.

7. The method according to any of claims 1 to 6, wherein the pressure of step (c) is between 1 bar and 1.5 bar.

8. A method for odourising a gas comprising the following steps:
i. producing the THT according to the method described in claims 1 to 7; and
ii. mixing the THT obtained in (i) with a gas selected from natural gas, biomethane and hydrogen.
